# EUROPEAN PATENT SPECIFICATION

(11) **EP 0 313 173 B1**
(45) Date of publication and mention of the grant of the patent: **16.07.1997**
(21) Application number: 88202362.5
(22) Date of filing: 21.10.1988
(51) Int. Cl.: A61B 5/04

(54) **Electrode assembly as well as method for producing same**
Elektrodenanordnung und Verfahren zu ihrer Herstellung
Ensemble d'électrodes et méthode de production dudit ensemble

(30) Priority: 23.10.1987 NL 8702534
(43) Date of publication of application: 26.04.1989
(73) Proprietor: Sippens Groenewegen, Arne, San Carlos, CA 94070-4521 (US)
(72) Inventor: Spekhorst, Johannes Hendrikus Maria, NL-1093 GE Amsterdam (NL); Sippens Groenewegen, Arne, NL-1054 XJ Amsterdam (NL)
(74) Representative: Wiegerink, Franciscus Johannes

(56) References cited:
- EP-A- 0 096 330
- WO-A-87/05814
- DE-A- 1 466 919
- NL-A- 7 012 672
- US-A- 4 102 331
- US-A- 4 109 648

## Description

This invention relates to an assembly comprising an electrode and a conducting wire attached thereto according to the preamble of claim 1 and to a method for producing same.

Such an electrode assembly is known from US-A-4 109 648 and may be used for investigation of the heart where the electric current generated by the heart muscle is used, which current may be measured on the body surface. The registration thereof gives an electro-cardiogram (ECG) and may serve for diagnostic or control purposes. The positions on the body where the current is measured have been standardized: the chest, arms and legs. During catheterization of the heart a catheter is positioned precisely by means of an intermitting X-ray view of the chest. In addition to the information of said X-ray view the cardiologist has the ECG at his disposal in order to judge the status of the heart. Since the known metal containing electrodes interfere with the X-ray view, such electrodes are not placed on the chest, but only on the arms and on the legs, i.e. outside the X-ray view. On the other hand, the so-called X-ray transparent electrodes may be placed on the chest. In this case the electrode material consists e.g. of thermoplastic polyester reinforced with carbon fibres. This material has a good electric conductivity. The connecting wire consists of carbon fibres and is also electro-conductive and X-ray transparent.

US patent specification 4,109,648 discloses an electrode assembly, the electrode comprising a hydrogel including carbon. Said hydrogel consists of a considerable amount of water e.g. 70% by wt., and a polymer forming a hydrophylic gel, e.g. hydroxyethyl methacrylate. The connecting wire may consist of carbon fibres. Said carbon fibres may be embedded in said hydrogel by polymerization of the starting materials of said hydrogel around the free end of the connecting wire which serves for attachment to the electrode.

On the other hand, it is generally known to attach connecting wires and electrodes by means of small clips,small plugs, snap fasteners and conductive glue.

The known X-ray transparent electrode assemblies have considerable disadvantages. Thus, the electrical properties (in particular the impedance) is often unsatisfactory and/or the attachment of the connecting wire with the electrode is vulnerable.

The aim of this invention is to abolish the abovementioned disadvantages.

This invention relates to an assembly comprising an electrode of conducting material and a conducting wire attached thereto, wherein the end of said conducting wire serving for the attachment to said electrode is present in the electrode material, wherein the assembly is X-ray transparant, the electrode consists of thermoplastic conducting material, and the end of the conducting wire being in contact with the electrode has the shape of a fanned-out bundle. Preferably, the electrode material consists of carbon containing plastic material, e.g. polyester, and the connecting wire consists of carbon fibres.

In the electrode assembly according to the invention the end of the conducting wire, which serves for attachment to the electrode, may be present in the electrode material in an arbitrary way. In the case of a conducting wire consisting of fibres, said fibres may be present as a bundle in said thermoplastic electrode material. In this case acceptable electrical properties are obtained. Optimal electrical properties are obtained, however, if the end of the conducting wire which serves for the attachment, is in contact with said electrode material through a surface area as large as possible.

The present invention also relates to a method for producing an assembly comprising an electrode of conducting thermoplastic material and a conducting wire attached thereto, said method being characterized in that the end of the conducting wire, which serves for the attachment, is introduced in the plasticized electrode material. Then said wire may be put into the molten electrode, e.g. pressed into the heated surface layer of the electrode. However, in a large-scale production it will be preferred to inject electrode material into a mold containing the end of said conducting wire.

In the method according to the invention it is preferred to arrange the end of said conducting wire before the introduction into or the application of the electrode material in such a form, that a surface as large as possible of the end of the wire is in contact with said electrode material. By this the electrical properties of the electrode are optimized. In the case of a conducting wire consisting of (carbon) fibres the fibres will be fanned out, for instance.

The invention also relates to an electrode assembly obtainable according to the method as described above.

Up to now, only disposable X-ray transparent electrodes have been commercially available. However, the mechanical quality of the electrode assembly according to invention is so good, that it may be used many times. The quality of the signal of the electrode assembly according to the invention may be compared with that of the classical silver/silver chloride electrode. The electrical properties are better than those of the commercially available X-ray transparent electrodes.

In the accompanying drawings embodiments of an electrode assembly according to the invention is further elucidated.

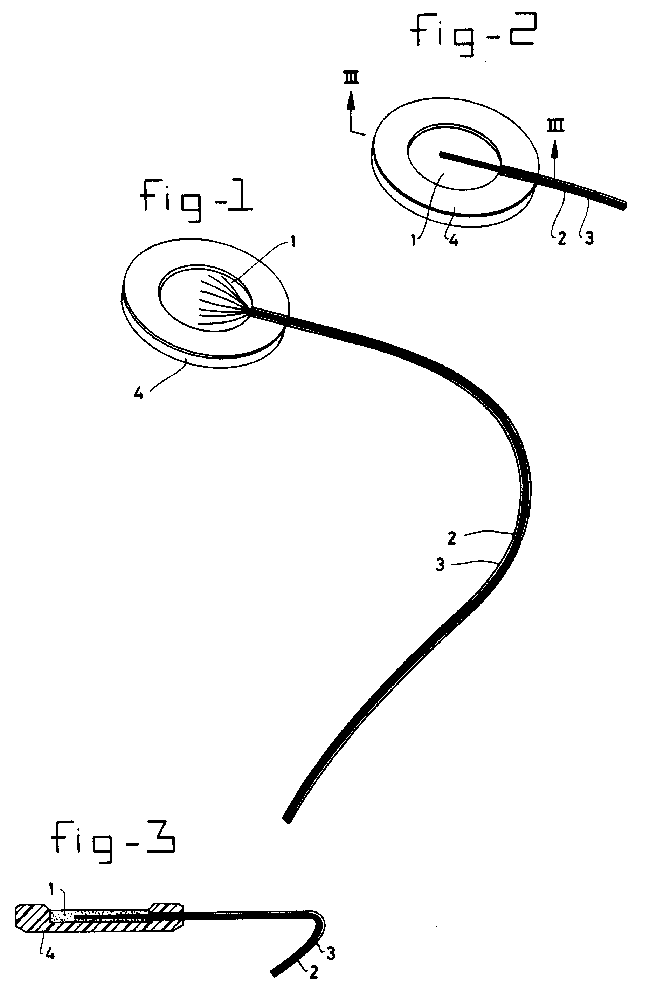

Figure 1 shows a view of an electrode assembly according to the invention. A disc-like, carbon containing plastic body 1 which may have a diameter of some millimeters up to some centimeters, contains the end of a conducting wire 2 in the shape of a fanned out bundle. The conducting wire 2 is provided with an electrical insulation 3 and the electrode body 1 is present in a housing 4 made of plastic. The edge of the housing 4 forms a cavity with the body 1. During the measurement on a patient a conducting paste is put into this cavity. In this way a more stable signal is obtained when the patient moves. All of the used materials are X-ray transparent.

Figure 2 also shows a view of an electrode assembly according to the invention; the reference numbers have the above indicated meaning. The difference from the embodiment according to figure 1 is that the end of the conducting wire has not been fanned out.

Figure 3 shows a cross sectional view along the line III-III of the embodiment according to figure 2.

It should be understood that the above described electrode assemblies and methods only comprise exemplary embodiments and that any modification obvious to an expert can be made therein without getting outside the scope of the present invention.

## Claims

1. Assembly comprising an electrode (1) of conducting material and a conducting wire (2) attached thereto, wherein the end of said conducting wire (2) serving for the attachment to said electrode (1) is present in the electrode material, characterised in that the assembly is X-ray transparant, the electrode (1) consists of thermoplastic conducting material, and that the end of the conducting wire (2) being in contact with the electrode (1) has the shape of a fanned-out bundle.

2. The assembly according to claim 1, characterised in that the electrode material consists of carbon containing plastic material and the conducting wire (2) consists of carbon fibres.

3. The assembly according to claim 1 or 2, characterised in that the end of the conducting wire (2) serving for the attachment to the electrode (1) is in contact with the electrode material through a surface area as large as possible.

4. A method for producing an assembly according to claim 1 to 3 and characterised in that the end of the conducting wire (2), which serves for the attachment to the electrode (1), is introduced into the plasticized electrode material.

5. A method for producing an assembly as defined in claims 1 to 3, characterised in that the electrode material is injected into a mold containing the end of the conducting wire (2).

## Patentansprüche

1. Baugruppe mit einer Elektrode (1) aus leitendem Material und einem daran befestigten leitenden Draht (2), wobei das der Befestigung an der Elektrode (1) dienende Ende des leitenden Drahtes (2) im Elektrodenmaterial vorliegt, dadurch gekennzeichnet, daß die Baugruppe Röntgenstrahlen durchläßt, die Elektrode (1) aus einem thermoplastischen leitenden Material besteht und daß das mit der Elektrode (1) in Kontakt stehende Ende des leitenden Drahtes (2) die Form eines aufgefächerten Bündels aufweist.

2. Baugruppe nach Anspruch 1, dadurch gekennzeichnet, daß das Elektrodenmaterial aus Kohlenstoff enthaltendes Kunststoffmaterial besteht und der leitende Draht (2) aus Kohlefasern besteht.

3. Baugruppe nach Anspruch 1 oder 2, dadurch gekennzeichnet, daß das der Befestigung an der Elektrode (1) dienende Ende des leitenden Drahtes (2) mit dem Elektrodenmaterial über eine größtmögliche Fläche in Kontakt steht.

4. Verfahren zur Herstellung einer Baugruppe nach Ansprüchen 1 bis 3 und dadurch gekennzeichnet, daß das Ende des leitenden Drahtes (2), das der Befestigung an der Elektrode (1) dient, in das plastifizierte Elektrodenmaterial eingeführt wird.

5. Verfahren zur Herstellung einer Baugruppe, wie in Ansprüchen 1 bis 3 definiert, dadurch gekennzeichnet, daß das Elektrodenmaterial in eine das Ende des leitenden Drahtes (2) enthaltende Form eingespritzt wird.

## Revendications

1. Assemblage comprenant une électrode (1) en une matière conductrice et un fil conducteur (2) fixé à l'électrode, dans lequel l'extrémité dudit fil conducteur (2) servant à sa fixation à ladite électrode (1) est présente dans la matière d'électrode, caractérisé en ce que l'assemblage laisse passer les rayons X et l'électrode (1) est constituée d'une matière conductrice thermoplastique et en ce que l'extrémité du fil conducteur (2) en contact avec l'électrode (1) a la forme d'un faisceau déployé en éventail.

2. Assemblage selon la revendication 1, caractérisé en ce que la matière d'électrode est constituée par une matière plastique carbonée et le fil conducteur (2) est constitué par des fibres de carbone.

3. Assemblage selon la revendication 1 ou 2, caractérisé en ce que l'extrémité du fil conducteur (2) servant à sa fixation à l'électrode (1) se trouve en contact avec la matière d'électrode via une aire de surface aussi grande que possible.

4. Procédé pour fabriquer un assemblage selon les revendications 1 à 3 et caractérisé en ce que l'extrémité du fil conducteur (2), qui sert à sa fixation à l'électrode (1), est introduite dans la matière d'électrode plastifiée.

5. Procédé pour fabriquer un assemblage selon les revendications 1 à 3, caractérisé en ce que la matière d'électrode est injectée dans un moule contenant l'extrémité du fil conducteur (2).
